(19) Europäisches Patentamt

European Patent Office

Office européen des brevets

(11) Veröffentlichungsnummer: **0 573 812 A2**

(12) **EUROPÄISCHE PATENTANMELDUNG**

(21) Anmeldenummer: **93107948.7**

(22) Anmeldetag: **15.05.93**

(51) Int. Cl.5: **C12N 5/00**

(30) Priorität: **12.06.92 DE 4219250**

(43) Veröffentlichungstag der Anmeldung:
**15.12.93 Patentblatt 93/50**

(84) Benannte Vertragsstaaten:
**AT BE CH DE DK ES FR GB GR IE IT LI LU NL PT SE**

(71) Anmelder: **BEHRINGWERKE Aktiengesellschaft**
**Postfach 1140**
**D-35001 Marburg(DE)**

(72) Erfinder: **Bosslet, Klaus**
**An der Haustatt 64**
**W-3550 Marburg(DE)**

(54) **Serum-freies Zellkulturmedium.**

㉗ Die Erfindung betrifft ein Nährmedium zur Vermehrung von Zellen, das Aminosäuren, Vitamine, Cofaktoren und anorganische Salze enthält, aber frei ist von tierischen Proteinen.

EP 0 573 812 A2

Die Erfindung betrifft ein Nährmedium zur Vermehrung von Zellen, das Aminosäuren, Vitamine, Cofaktoren und anorganische Salze enthält, aber frei ist von tierischen Proteinen.

Die Herstellung moderner Pharmaka auf biotechnologischer Basis ist in vielen Fällen auf komplizierte eukaryontische Zellkulturproduktionssysteme angewiesen, da viele Glykoproteine nicht funktionell aktiv in einfachen Prokaryonten hergestellt werden können. Diese komplexen eukaryontischen Zellkultursysteme benötigen Nährmedien, die neben definierten Salzen, Vitaminen, Cofaktoren und Aminosäuren (Basalmedium) eine Mischung hochmolekularer Proteine oder Lipide enthalten, besonders das teure fötale Rinderserum (fetal bovine serum; FBS). Diese in großer Zahl und hoher Konzentration im Nährmedium benötigten, aber schlecht definierten, hochmolekularen, beispielsweise aus fötalem Rinderserum stammenden Bestandteile müssen über aufwendige Reinigungsverfahren nach der Fermenterproduktionsphase vom herzustellenden Pharmakum abgetrennt werden. Des weiteren muß sichergestellt sein, daß das verwendete FBS keine pathogenen Erreger enthält.

Wünschenswert wäre es, ein billiges Nährmedium zu haben, welches das Wachstum einer breiten Palette eukaryontischer Zellinien ebensogut ermöglicht wie teures FBS-haltiges Nährmedium und welches keine tierischen Fremdproteine enthält.

Überraschenderweise wurde gefunden, daß es möglich ist, ein definiertes, kostengünstiges Nährmedium herzustellen, welches das permanente Wachstum einer Vielzahl eukaryontischer Zellinien, vergleichbar gut wie FBS-haltiges Nährmedium, erlaubt. Es enthält keine animalen Proteine, sondern außer Aminosäuren, Vitaminen, Cofaktoren und üblichen anorganischen Salzen humanes Transferrin, humanes Insulin, Ethanolamin und Natriumselenit.

Gegenstand der Erfindung ist ein Nährmedium für Zellkulturen enthaltend Aminosäuren, Vitamine, Cofaktoren und übliche anorganische Salze, dadurch gekennzeichnet, daß es humanes Transferrin, humanes Insulin, Ethanolamin und Natriumselenit enthält.

Vorzugsweise werden Klinisch zugelassenes Transferrin und Insulin verwendet.

Als Medium, das Aminosäuren, Vitamine, Cofaktoren und anorganische Salze (Basalmedium) enthält, kann eines der im Stand der Technik bekannten Medien eingesetzt werden.

Solche Grundmedien sind bekannt, beispielsweise das MEM Alpha Medium, DMEM/F 12(1:1) Medium, DMEM (Dulbecco's Modifiziertes Eagle Medium), William's Medium E, Ham's F12 Medium.

Weitere Grundmedien können verwendet werden, wenn Natrium-Pyruvat in der Konzentration 25-500, vorzugsweise 110 ng/l zugegeben wird. Diese Grundmedien sind beispielsweise: RPMI-1640 Medium, CMRL-1066 Medium, BME-Basalmedium, Waymouth's Medium, NCTC-135 Medium, MEM (Minimum Essential Medium).

Zu einem dieser Grundmedien werden Insulin und Transferrin zugegeben, so daß eine Konzentration von 6-20, vorzugsweise 8-12 mg/l Insulin und 20-50, vorzugsweise 30-40 mg/l Transferrin im Medium erreicht wird.

Die Zugabe von Ethanolamin und Natriumselenit zu dem Grundmedium erfolgt so, daß eine Konzentration von 1.3-20, vorzugsweise 8-14 mg/l Ethanolamin, und 2-20, vorzugsweise 6-10 $\mu$g/l, Natriumselenit im Medium erreicht wird.

Dem Medium wird vor Gebrauch L-Glutamin zugesetzt, so daß eine Konzentration an dieser Aminosäure von 140-1200 mg/l, vorzugsweise 600 mg/l erreicht wird.

**Beispiel:**

Es wurde ein Medium mit folgender Zusammensetzung hergestellt. Die Mengen (in Gramm) beziehen sich auf 200 Liter Medium.

2

| Grundmediumkomponenten: | |
|---|---|
| NaCl | 1 280,00 |
| KCl | 80,00 |
| $MgSO_4 \cdot 7 H_2O$ | 40,00 |
| $NaH_2PO_4 \cdot H_2O$ | 25,00 |
| $CaCl_2 \cdot 2 H_2O$ | 52,80 |
| $Fe(NO_3)_3 \cdot 9 H_2O$ | 0,02 |
| Phenolrot | 3,70 |
| $Glucose \cdot H_2O$ | 990,00 |
| Brenztraubensäure-Natriumsalz | 22,10 |
| L-Alanin | 7,14 |
| L-Arginin-HCl | 16,80 |
| $L-Asparagin \cdot H_2O$ | 13,66 |
| L-Asparaginsäure | 10,63 |
| L-Cystein | 9,60 |
| L-Glutaminsäure | 11,83 |
| Glycin | 6,02 |
| $L-Histidin-HCl \cdot H_2O$ | 8,40 |
| L-Isoleucin | 21,00 |
| L-Leucin | 21,00 |
| L-Lysin-HCl | 29,20 |
| L-Methionin | 6,00 |
| L-Phenylalanin | 13,20 |
| L-Prolin | 9,22 |
| L-Serin | 8,42 |
| L-Threonin | 19,00 |
| L-Tryptophan | 3,20 |
| L-Tyrosin | 14,40 |
| L-Valin | 18,80 |
| Calciumpantothenat | 0,80 |
| Cholinchlorid | 0,80 |
| Folsäure | 0,80 |
| I-Inosit | 1,44 |
| Nicotinsäureamid | 0,80 |
| Pyridoxal-HCl | 0,80 |
| Riboflavin | 0,08 |
| Thiamin-HCl | 0,80 |
| $NaHCO_3$ | 750,00 |

| Zusatzkomponenten: | |
|---|---|
| Human-Insulin (Hoechst:PHFM) | 2,00 |
| Human-Transferrin (Behringwerke AG, Produktbezeichnung: Ø TRE) | 7,00 |
| Ethanolamin | 1,68 ml |
| Natriumselenit (1 mM) | 9,4 ml |
| Aqua-Dest. | ad 200 l |

Nach Zusatz von 600 mg/l Glutamin wurden Hybridome und Transfektome in diesem Medium und im Vergleich dazu in FBS-haltigem Grundmedium (Dulbeccos Medium + FBS) vermehrt. Das Ergebnis ist in Tabelle I gezeigt. Die Zellen wurden unter Standardzellkulturbedingungen bei 37 °C und 5% $CO_2$ in Dampf-gesättigter Atmosphäre über die in der Tabelle I vermerkten Zeiträume kultiviert und aus Aliquots die Lebendzellzahl bzw. die Produktkonzentration bestimmt.

Die Daten belegen die Gleichwertigkeit des DKHI-Mediums in bezug auf Zellwachstum und Produktionsleistung gegenüber dem D-FBS Vollmedium. Die Vermehrungsraten sind für beide Medien gleich.

Weiterhin konnten Humane und Ratten- Tumorzellinien in dem Medium kultiviert werden (Tabelle II). Die Vergleichsmedien waren DMEM + 10 % FBS und ein von Murakami et al. (Proc. natl. Acad. Sci. USA 79, 1158-1162, 1982) publiziertes ITES-Medium.

Das DKHI-Medium unterscheidet sich von dem ITES-Medium dadurch, daß es 10 mg/l Insulin, 200 µMol/l Ethanolamin, 1 mMol/l Brenztraubensäure und 3.75 g Natriumhydrogenkarbonat/l enthält, sowie keinen HEPES Puffer (siehe Tabelle III, zum Vergleich). Diese Unterschiede führen zu einem verbesserten Zellwachstum, wie in Tabelle II dokumentiert.

## Tabelle I

| Zellinie | Zellzahl x 10⁵/ml am Tag xxx D-FBS/DKHI (5 Ansätze) | | | | | Produktionsleistung in ng Protein/ml am Tag D-FBS/DKHI |
|---|---|---|---|---|---|---|
| **Transfektome auf BHK-Basis** | | | | | | |
| B 70/6 | 2/2 | 2/2 | 4/4 | 8/8 | 8/8 | 1000-2000 / 500-2000 x |
| B 52/44 | 3/3 | 3/3 | 6/6 | 10/10 | 10/10 | 1000-2500 / 1000-2000 x |
| B 101/8 | 2/2 | 2/2 | 4/4 | 8/8 | 8/8 | 2000-3000 / 2500-4000 x |
| **Hybridome auf SP-2 Basis** | | | | | | |
| 835/6 | 2/2 | 3/3 | 6/6 | 12/12 | 12/12 | 12000/30000 xx |
| 2128/65 | 2/2 | 3/3 | 6/6 | 12/12 | 12/12 | 10000/ 2000 xx |
| **NSO Basis** | | | | | | |
| 2064/34 | 2/2 | 3/3 | 6/6 | 10/10 | 10/10 | 20000/20000 xx |
| 2093/1072 | 2/2 | 3/3 | 6/6 | 11/11 | 11/11 | 20000/25000 xx |

x: humanes IgG
xx: Maus IgG
xxx: bestimmt in einer mikroskopischen Zählkammer

EP 0 573 812 A2

## Tabelle II

| | DKHI | DMEM + 10 % FBS | ITES |
|---|---|---|---|
| Mamma Ca (Human): T-47 | Wachstum | Wachstum | kein Wachstum |
| Melanom (Human): M-21 | - " - | - " - | - " - |
| Pankreas Ca (Human): PaTu 8902 | - " - | - " - | - " - |
| MiaPaCa 2 | - " - | - " - | - " - |
| Colon Ca (Human): LOVO | - " - | - " - | - " - |
| LS-174-T | - " - | - " - | - " - |
| Magen Ca (Human): Mz-Sto-1 | - " - | - " - | - " - |
| Mamma Adeno Ca (Ratte): Rc3230Ac | - " - | - " - | - " - |
| Makrophagen (Human): U-937 | - " - | - " - | - " - |

Tabelle III

| Vergleich der Zusatzkomponenten des ITES Mediums und des DKHI Mediums | | |
|---|---|---|
| | ITES | DKHI |
| Grundmedium | DMEM/F12 (1:1) | DMEM |
| HEPES | 15 mM | - * |
| NaHCO$_3$ | 1,2 g/l | 3,75 g/l * |
| Insulin | 5 mg/l | 10 mg/l * |
| Transferrin | 35 mg/l | 35 mg/l |
| Na-Selenit | 2,5 nM - 2,5 mM | 50 nM |
| Ethanolamin | 20 $\mu$M | 200 $\mu$M * |
| Na-Pyruvat | - | 1 mM * |
| L-Glutamin | - | 2 mM * |

\* signifikante Unterschiede zwischen ITES Medium und DKHI Medium

## Patentansprüche

1. Nährmedium für Zellkulturen enthaltend ein Grundmedium enthaltend Aminosäuren, Vitamine, Cofaktoren und übliche anorganische Salze, dadurch gekennzeichnet, daß es zusätzlich humanes Transferrin, humanes Insulin, Ethanolamin und Natriumselenit enthält.

2. Nährmedium nach Anspruch 1, dadurch gekennzeichnet, daß es Klinisch zugelassenes Transferrin und Insulin enthält.

3. Nährmedium nach Anspruch 1, dadurch gekennzeichnet, daß als Grundmedium DMEM/F 12(1:1) Medium, DMEM (Dulbecco's Modifiziertes Eagle Medium), William's Medium E oder Ham's F12 Medium verwendet wird.

4. Nährmedium nach Anspruch 1, dadurch gekennzeichnet, daß als Grundmedium RPMI-1640 Medium, CMRL-1066 Medium, BME-Basalmedium, Waymouth's Medium, NCTC-135 Medium oder MEM (Minimum Essential Medium) verwendet und Natrium Pyruvat in einer Menge zugegeben wird, daß eine Konzentration von 25-500, vorzugsweise 110 mg/l Natrium-Pyruvat im Medium erreicht wird.

5. Nährmedium nach Anspruch 1, dadurch gekennzeichnet, daß Insulin in einer Menge zugegeben wird, daß eine Konzentration von 6-20, vorzugsweise 8-12 mg/l Insulin im Medium erreicht wird.

6. Nährmedium nach Anspruch 1, dadurch gekennzeichnet, daß Transferrin in einer Menge zugegeben wird, daß eine Konzentration von 20-50, vorzugsweise 30-40 mg/l Transferrin im Medium erreicht wird.

7. Nährmedium nach Anspruch 1, dadurch gekennzeichnet, daß Ethanolamin in einer Menge zugesetzt wird, daß eine Konzentration von 1,3-20, vorzugsweise 10-14 mg/l Ethanolamin im Medium erreicht wird.

8. Nährmedium nach Anspruch 1, dadurch gekennzeichnet, daß Natriumselenit in einer Menge zusetzt wird, daß eine Konzentration von 2-20, vorzugsweise 6-10 $\mu$g/l Natriumselenit im Medium erreicht wird.

9. Nährmedium nach Anspruch 1, dadurch gekennzeichnet, daß L-Glutamin in einer Menge zugesetzt wird, daß eine Konzentration von 140-1200, vorzugsweise 600 mg/l L-Glutamin im Medium erreicht wird.

10. Nährmedium nach Anspruch 1, dadurch gekennzeichnet, daß es in 200 Liter Flüssigkeit folgende Bestandteile (in Gramm) enthält:

| Grundmediumkomponenten: | |
| --- | --- |
| NaCl | 1 280,00 |
| KCl | 80,00 |
| $MgSO_4 . 7 H_2O$ | 40,00 |
| $NaH_2PO_4 . H_2O$ | 25,00 |
| $CaCl_2 . 2 H_2O$ | 52,80 |
| $Fe(NO_3)_3 . 9 H_2O$ | 0,02 |
| Phenolrot | 3,70 |
| $Glucose . H_2O$ | 990,00 |
| Brenztraubensäure-Natriumsalz | 22,10 |
| L-Alanin | 7,14 |
| L-Arginin-HCl | 16,80 |
| $L\text{-Asparagin} . H_2O$ | 13,66 |
| L-Asparaginsäure | 10,63 |
| L-Cystein | 9,60 |
| L-Glutaminsäure | 11,83 |
| Glycin | 6,02 |
| $L\text{-Histidin-HCl} . H_2O$ | 8,40 |
| L-Isoleucin | 21,00 |
| L-Leucin | 21,00 |
| L-Lysin-HCl | 29,20 |
| L-Methionin | 6,00 |
| L-Phenylalanin | 13,20 |
| L-Prolin | 9,22 |
| L-Serin | 8,42 |
| L-Threonin | 19,00 |
| L-Tryptophan | 3,20 |
| L-Tyrosin | 14,40 |
| L-Valin | 18,80 |
| Calciumpantothenat | 0,80 |
| Cholinchlorid | 0,80 |
| Folsäure | 0,80 |
| I-Inosit | 1,44 |
| Nicotinsäureamid | 0,80 |
| Pyridoxal-HCl | 0,80 |
| Riboflavin | 0,08 |
| Thiamin-HCl | 0,80 |
| $NaHCO_3$ | 750,00 |

| Zusatzkomponenten: | |
| --- | --- |
| Human-Insulin (Hoechst:PHFM) | 2,00 |
| Human-Transferrin (Behringwerke AG, Produktbezeichnung: Ø TRE) | 7,00 |
| Ethanolamin | 1,68 ml |
| Natriumselenit (1 mM) | 9,4 ml |
| Aqua-Dest. | ad 200 l |

und vor Gebrauch wird L-Glutamin in einer Menge zugegeben, daß eine Konzentration von 140-1200 mg/l, vorzugsweise 600 mg/l, erreicht wird.

**11.** Verwendung eines Nährmediums nach Anspruch 1 zur Vermehrung von Hybridomen, Transfektomen oder Tumorzellinien.